# EUROPEAN PATENT APPLICATION

(11) **EP 4 162 939 A1**
(43) Date of publication of application: **12.04.2023**
(21) Application number: 21817674.1
(22) Date of filing: 02.06.2021
(51) Int. Cl.: A61K 31/4164, A61P 17/00, A61P 17/06, A61P 27/16, A61P 31/10

(54) **ANTIFUNGAL AGENT FOR USE IN HUMANS**

(30) Priority: 03.06.2020 JP 2020097174
(71) Applicant: Ishihara Sangyo Kaisha, Ltd., Osaka-shi, Osaka 550-0002 (JP)
(72) Inventor: SHIKAMA Hiroshi, Osaka-shi, Osaka 550-0002 (JP); HIGUCHI Koji, Osaka-shi, Osaka 550-0002 (JP); ATSUMI Shogo, Osaka-shi, Osaka 550-0002 (JP); IMURA Takayuki, Osaka-shi, Osaka 550-0002 (JP)
(74) Representative: Regimbeau
(86) International application number: PCT/JP2021/021064
(87) International publication number: WO 2021/246455

(57) **Abstract**

A novel antifungal agent for a human is provided, and a compound represented by formula (I) or (II) or a salt thereof is used as an antifungal agent for a human.

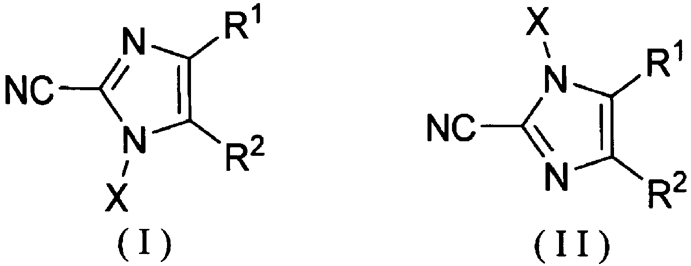

## Description

### Cross-reference to related application

The present patent application claims priority based on Japanese Patent Application No. 2020-097174 filed on June 3, 2020, and the contents of this prior patent application are incorporated herein by reference in its entirety.

### Technical Field

A microbe belonging to the genus Malassezia is a basidiomycetous yeast that resides in the skin of a human or an animal, and the microbe proliferates using a lipid as a nutrient source and thus settles in an area having a lot of sebum and is known to cause a skin disease such as tinea versicolor, seborrheic dermatitis, folliculitis, atopic dermatitis, or psoriasis vulgaris.

Examples of a conventional therapeutic agent for a skin disease derived from a microbe belonging to the genus Malassezia include selenium disulfide, zinc 2-mercaptopyridine N-oxide, piroctone olamine, an imidazole-based compound such as miconazole nitrate or ketoconazole, and a triazole-based compound such as itraconazole or fluconazole.

Usually, a normal skin inhabitant has a so-called barrier effect such as the effect of preventing the scalp odor associated with the growth of various germs on the skin or the effect of keeping a weakly acidic state by moderately degrading sebum, but a conventional therapeutic agent kills not only a pathogenic microbe but also a normal skin inhabitant, and thus there is a concern that the barrier effect of the skin may be destroyed. Further, it has been pointed out that selenium disulfide has a side effect such as oral toxicity, hair loss, skin irritation, debility, malaise, or discoloration of hair, and zinc 2-mercaptopyridine N-oxide has a concern about a sulfurous odor or an endocrine disruptor. Further, an imidazole-based compound such as miconazole nitrate and a triazole compound such as itraconazole have the action of inhibiting the lipid synthesis of a microbe, but because of the property of developing antimicrobial activity by inhibiting the biosynthesis of the cell membrane of a microbe, these compounds take time to develop a sufficient effect and need to be administered for a long period of time. It is known that consecutive administration of the same agent over a long period of time greatly increases the risk of developing a resistant microbe.

As described above, a lipid synthesis inhibitor does not immediately develop antimicrobial activity even when this agent is administered, and thus is not suitable for an administration method that requires immediate onset of antimicrobial activity, for example, the prevention or the treatment of a skin disease using a medical shampoo. Therefore, there has been a demand for the development of a compound which is highly safe, has a mechanism of action different from that of a conventional therapeutic agent, and exerts antimicrobial activity immediately.

For example, Non-Patent Document 1 discloses that the mechanism of action of cyazofamid, which is a cyanoimidazole-based compound, is respiratory inhibition.

Patent Document 1 discloses that an imidazole-based compound including cyazofamid is useful as a pest control agent, and Patent Document 2 discloses that a control agent containing an imidazole-based compound including cyazofamid as an active component is useful against an animal disease derived from a parasite, for example a parasite such as a coccidium. However, nothing has been reported about the antimicrobial activity of the imidazole-based compound against a skin fungus involved in a human disease such as cutaneous malasseziosis, a microbe belonging to the genus Candida, a microbe belonging to the genus Malassezia, a microbe belonging to the genus Trichophyton, a microbe belonging to the genus Microsporum, a microbe belonging to the genus Arthroderma, a microbe belonging to the genus Aspergillus, or a microbe belonging to the genus Cryptococcus.

### Piror Art

Patent Document 1: Japanese Patent Laid-Open No. H1-131163
Patent Document 2: International Publication No. WO 01/14341
Non-Patent Document 1: Pesticide Biochemistry and Physiology, 2001, vol.71: 107-115

### Summary of the Invention

The present disclosers have found that a cyanoimidazole-based compound represented by formula (I) or (II) described later (hereinafter, also referred to as a compound of the present disclosure) can be used as a novel antifungal agent for a human that is effective against a skin fungus involved in a human disease.

Therefore, the present disclosure provides a novel antifungal agent for a human.

That is, the present disclosure provides an antifungal agent for a human comprising a compound represented by formula (I) or (II) or a salt thereof. 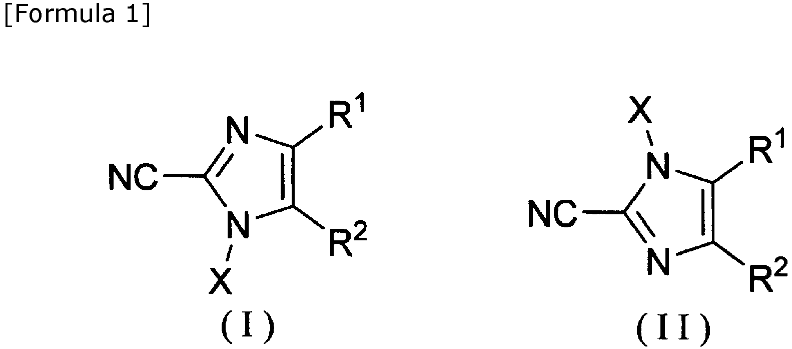 wherein
R¹ and R² are each independently
a hydrogen atom,
a halogen atom,
a hydroxyl group,
a nitro group,
a cyano group,
a thiocyanate group,
a trimethylsilyl group,
an optionally substituted alkyl group,
an optionally substituted alkenyl group,
an optionally substituted alkynyl group,
an optionally substituted alkyloxy group,
an optionally substituted alkenyloxy group,
an optionally substituted alkynyloxy group,
an optionally substituted aryl group,
an optionally substituted aryloxy group,
an optionally substituted 5- or 6-membered aromatic heterocyclic group,

   -SOₘR³
wherein R³ is an optionally substituted alkyl group, an optionally substituted alkenyl group, an optionally substituted alkynyl group, an optionally substituted aryl group, an optionally substituted 5- or 6-membered aromatic heterocyclic group, or a -NR⁴R⁵ group wherein R⁴ and R⁵ are each an optionally substituted alkyl group, and m is an integer of 0 to 2, or
a group represented by 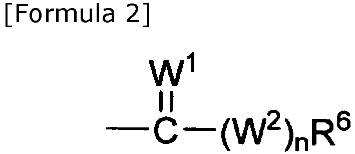
wherein
W¹ is an oxygen atom or a sulfur atom,
W² is an oxygen atom, a sulfur atom, or -NH-
n is an integer of 0 or 1,
R⁶ is an optionally substituted alkyl group or an optionally substituted aryl group;
X is a hydrogen atom, a hydroxyl group, or -OY; and
Y is a formula: 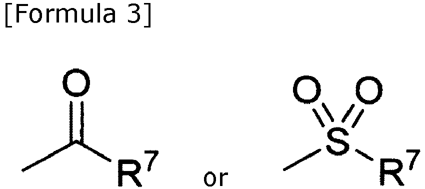
wherein
R⁷ is an optionally substituted alkyl group,
an optionally substituted alkyloxy group,
an optionally substituted alkenyl group,
an optionally substituted alkenyloxy group,
an optionally substituted aryl group,
an optionally substituted aryloxy group,
an optionally substituted 5- or 6-membered aromatic heterocyclic group,
a -NR⁸R⁹ group wherein R⁸ and R⁹ are each independently a hydrogen atom, an optionally substituted alkyl group, or an optionally substituted alkenyl group, or together with a nitrogen atom adjacent to these, form a 5- to 7-membered saturated heterocycle, unless R⁸ and R⁹ are hydrogen atoms at the same time, or
a -CR¹⁰R¹¹R¹² group wherein R¹⁰, R¹¹, and R¹² are each independently an optionally substituted alkyl group, an optionally substituted alkenyl group, or an optionally substituted aryl group.

According to the present disclosure, it is possible to provide a novel antifungal agent for a human by using a compound represented by formula (I) or (II) or a salt thereof. The compound represented by formula (I) or (II) or a salt thereof can be advantageously used for immediately exerting excellent antimicrobial activity against a fungus associated with a human disease.

### Detailed Description of the Invention

As used herein, "halogen" means fluorine, chlorine, bromine, or iodine, and is preferably fluorine, chlorine, or bromine.

Further, as used herein, the term "alkyl," "alkenyl," or "alkynyl" as a group or a part of a group means alkyl, alkenyl, or alkynyl, respectively, where the group is linear, branched chain, cyclic, or a combination thereof, unless otherwise defined. Further, for example, in the case of "alkyl having 1 to 6 carbon atoms" as a group or a part of a group, "1 to 6 carbon atoms" means that the alkyl group has 1 to 6 carbon atoms.

Further, as used herein, an alkyl group being "optionally substituted" means that one or more hydrogen atoms on the alkyl group may be replaced with one or more substituents (which may be the same or different). It will be apparent to those skilled in the art that the maximum number of substituents can be determined depending on the number of replaceable hydrogen atoms on the alkyl. The same also applies to a functional group other than an alkyl group.

When the alkyl group represented by any of R¹ to R¹² is linear or branched, the number of carbon atoms of the linear or branched alkyl group is preferably 1 to 12, more preferably 1 to 6, and even more preferably 1 to 3. Examples of the linear or branched alkyl group include a methyl group, an ethyl group, a n-propyl group, an isopropyl group, a n-butyl group, a sec-butyl group, an isobutyl group, a tert-butyl group, a n-pentyl group, a n-hexyl group, a heptyl group, an octyl group, a nonyl group, or a decyl group, and a methyl group, an ethyl group, a n-propyl group or an isopropyl group is preferable, and a methyl group or an ethyl group is more preferable.

When the alkyl group represented by any of R¹ to R¹² is cyclic, the number of carbon atoms of the cyclic alkyl group (cycloalkyl group) is preferably 3 to 7, and more preferably 3 to 6. Examples of the cycloalkyl group include a cyclopropyl group, a cyclobutyl group, a cyclopentyl group, or a cyclohexyl group.

The alkyl group is optionally substituted, and examples of the substituent include a halogen atom; an alkyloxy group optionally substituted with a halogen atom; an alkylthio group optionally substituted with a halogen atom; a phenyl group optionally substituted with a halogen atom; a phenyl group substituted with an alkyl group optionally substituted with a halogen atom; and a hydroxyl group. When the alkyl group is cyclic (cycloalkyl group), examples of the substituent include an alkyl group optionally substituted with a halogen atom in addition to the above substituents. The number of such substituents is preferably 0 to 5, and more preferably 1 or 2.

When the alkyloxy group represented by any of R¹, R², and R⁷ is linear or branched, the number of carbon atoms of the alkyloxy group is preferably 1 to 12, more preferably 1 to 6, and even more preferably 1 to 3. Examples of the linear or branched alkyloxy group include a methoxy group, an ethoxy group, a n-propyloxy group, an isopropyloxy group, a n-butyloxy group, a sec-butyloxy group, an isobutyloxy group, a tert-butyloxy group, a n-pentyloxy group, a n-hexyloxy group, a heptyloxy group, an octyloxy group, a nonyloxy group, or a decyloxy group, and a methoxy group, an ethoxy group, a n-propyloxy group, or an isopropyloxy group are preferable, and a methoxy group or an ethoxy group is more preferable.

When the alkyloxy group represented by any of R¹, R², and R⁷ is cyclic, the number of carbon atoms of the cyclic alkyloxy group (cycloalkyloxy group) is preferably 3 to 7, and more preferably 3 to 6. Examples of the cycloalkyloxy group include a cyclopropyloxy group, a cyclobutyloxy group, a cyclopentyloxy group, or a cyclohexyloxy group.

The alkyloxy group is optionally substituted, and examples of the substituent include a halogen atom; an alkyloxy group optionally substituted with a halogen atom; an alkylthio group optionally substituted with a halogen atom; a phenyl group optionally substituted with a halogen atom; a phenyl group substituted with an alkyl group optionally substituted with a halogen atom; and a hydroxyl group. When the alkyloxy group is cyclic (cycloalkyloxy group), examples of the substituent include an alkyl group optionally substituted with a halogen atom in addition to the above substituents. The number of such substituents is preferably 0 to 5, and more preferably 1 or 2.

When the alkenyl group represented by any of R¹, R², R³, R⁷, R¹⁰, R¹¹, and R¹² is linear or branched, the number of carbon atoms of the linear or branched alkenyl group is preferably 2 to 12, more preferably 2 to 6, and even more preferably 2 to 4. Examples of the linear or branched alkenyl group include an allyl group and a geranyl group.

When the alkenyl group represented by any of R¹, R², R³, R⁷, R¹⁰, R¹¹, and R¹² is cyclic, the number of carbon atoms of the cyclic alkenyl group (cycloalkenyl group) is preferably 5 to 8, and more preferably 5 or 6. Examples of the cycloalkenyl group include a cyclopentenyl group, a cyclohexenyl group, and a cyclooctenyl group.

The alkenyl group is optionally substituted, and examples of the substituent include a halogen atom; an alkyloxy group optionally substituted with a halogen atom; an alkylthio group optionally substituted with a halogen atom; a phenyl group substituted with a halogen atom; a phenyl group substituted with an alkyl group optionally substituted with a halogen atom; and a hydroxyl group. When the alkenyl group is cyclic (cycloalkenyl group), examples of the substituent include an alkyl group optionally substituted with a halogen atom in addition to the above substituents. The number of such substituents is preferably 0 to 5, and more preferably 1 or 2.

When the alkenyloxy group represented by any of R¹, R², and R⁷ is linear or branched, the number of carbon atoms of the linear or branched alkenyloxy group is preferably 2 to 12, more preferably 2 to 6, and even more preferably 2 to 4. Examples of the linear or branched alkenyloxy group include a 2-propenyloxy group.

When the alkenyloxy group represented by any of R¹, R², and R⁷ is cyclic, the number of carbon atoms of the cyclic alkenyloxy group (cycloalkenyloxy group) is preferably 5 to 8, and more preferably 5 or 6. Examples of the cycloalkenyloxy group include a cyclopentenyloxy group, a cyclohexenyloxy group, and a cyclooctenyloxy group.

The alkenyloxy group is optionally substituted, and examples of the substituent include a halogen atom; an alkyloxy group optionally substituted with a halogen atom; an alkylthio group optionally substituted with a halogen atom; a phenyl group optionally substituted with a halogen atom; a phenyl group substituted with an alkyl group optionally substituted with a halogen atom; and a hydroxyl group. When the alkenyloxy group is cyclic (cycloalkenyloxy group), examples of the substituent include an alkyl group optionally substituted with a halogen atom in addition to the above substituents. The number of such substituents is preferably 0 to 5, and more preferably 1 or 2. The number of such substituents is preferably 0 to 5, and more preferably 1 or 2.

When the alkynyl group represented by any of R¹, R², and R³ is linear or branched, the number of carbon atoms of the linear or branched alkynyl group is preferably 2 to 12, more preferably 2 to 6, and even more preferably 2 to 4. Examples of the linear or branched alkynyl group include a 2-propynyl group.

When the alkynyl group represented by any of R¹, R², and R³ is cyclic, the number of carbon atoms of the cyclic alkynyl group (cycloalkynyl group) is preferably 6 to 10. Examples of the cycloalkynyl group include a cyclooctynyl group.

The alkynyl group is optionally substituted, and examples of the substituent include a halogen atom; an alkyloxy group optionally substituted with a halogen atom; an alkylthio group optionally substituted with a halogen atom; a phenyl group optionally substituted with a halogen atom; a phenyl group substituted with an alkyl group optionally substituted with a halogen atom; and a hydroxyl group. When the alkynyl group is cyclic (cycloalkynyl group), examples of the substituent include an alkyl group optionally substituted with a halogen atom in addition to the above substituents. The number of such substituents is preferably 0 to 5, and more preferably 1 or 2.

When the alkynyloxy group represented by any of R¹ and R² is linear or branched, the number of carbon atoms of the linear or branched alkynyloxy group is preferably 2 to 12, more preferably 2 to 6, and even more preferably 2 to 4. Examples of the linear or branched alkynyloxy group include a 2-propynyloxy group.

When the alkynyloxy group represented by any of R¹ and R² is cyclic, the number of carbon atoms of the cyclic alkynyloxy group (cycloalkynyloxy group) is preferably 6 to 10. Examples of the cycloalkynyloxy group include a cyclooctynyloxy group.

The alkynyloxy group is optionally substituted, and examples of the substituent include a halogen atom; an alkyloxy group optionally substituted with a halogen atom; an alkylthio group optionally substituted with a halogen atom; a phenyl group optionally substituted with a halogen atom; a phenyl group substituted with an alkyl group optionally substituted with a halogen atom; and a hydroxyl group. When the alkynyloxy group is cyclic (cycloalkynyloxy group), examples of the substituent include an alkyl group optionally substituted with a halogen atom in addition to the above substituents. The number of such substituents is preferably 0 to 5, and more preferably 1 or 2.

The number of carbon atoms of the aryl group represented by any of R¹, R², R³, R⁶, R⁷, R¹⁰, R¹¹, and R¹² is preferably 6 to 14, and more preferably 6 to 10. Examples of the aryl group include a phenyl group and a naphthyl group, and a phenyl group is preferable.

The number of carbon atoms of the aryl moiety of the aryloxy group represented by any of R¹, R², R³, R⁶, R⁷, R¹⁰, R¹¹, and R¹² is preferably 6 to 14, and more preferably 6 to 10. Examples of the aryloxy group include a phenyloxy group and a naphthyloxy group, and a phenyloxy group is preferable.

The aryl group or the aryloxy group is optionally substituted, and examples of the substituent include a halogen atom; an alkyl group optionally substituted with a halogen atom; an alkyloxy group optionally substituted with a halogen atom; an alkylthio group optionally substituted with a halogen atom; a phenyl group optionally substituted with a halogen atom; a phenyl group substituted with an alkyl group optionally substituted with a halogen atom; and a hydroxyl group. The number of such substituents is preferably 0 to 5, and more preferably 1 or 2.

The 5- or 6-membered aromatic heterocyclic group represented by any of R¹, R², R³, and R⁷ is preferably an aromatic heterocycle having one or more heteroatoms selected from the group consisting of nitrogen, oxygen, and sulfur which are the same or different. The number of heteroatoms in the 5- or 6-membered aromatic heterocyclic group is preferably 1 to 4, more preferably 1 to 3, and even more preferably 1 or 2. Examples of the 5- or 6-membered aromatic heterocyclic group include a thienyl group, a furyl group, a thiazolyl group, and a pyridyl group.

The 5- or 6-membered aromatic heterocyclic group is optionally substituted, and examples of the substituent include a halogen atom, a nitro group, a cyano group, an alkyl group optionally substituted with a halogen atom, an alkyloxyalkyl group, an alkyloxy group optionally substituted with a halogen atom, a methylenedioxy group optionally substituted with a halogen atom, a -NR¹³R¹⁴ group wherein R¹³ and R¹⁴ are each a hydrogen atom, an alkyl group optionally substituted with a halogen atom, or an alkanoyl group, and a -SOpR¹⁵ group wherein R¹⁵ is an alkyl group optionally substituted with a halogen atom, and p is an integer of 0 to 2. The number of such substituents is preferably 0 to 5, and more preferably 1 or 2. The alkyl moiety included in any of R¹³ to R¹⁵ may be the same as the alkyl moiety in any of R¹ to R¹² described above.

The 5- to 7-membered saturated heterocycle formed by R⁸ and R⁹ groups together with a nitrogen atom adjacent to these is preferably a saturated heterocycle having one or more heteroatoms selected from the group consisting of nitrogen, oxygen, and sulfur which are the same or different. The number of heteroatoms in the 5- to 7-membered saturated heterocycle is preferably 1 to 4, more preferably 1 to 3, and even more preferably 1 or 2. Examples of the 5- to 7-membered saturated heterocycle include a piperidine group, a pyrrolidine group, a morpholine group, a thiomorpholine group.

According to a preferable embodiment of the present disclosure, R¹ and R² are each independently a hydrogen atom, a halogen atom, a hydroxyl group, a nitro group, a cyano group, a thiocyanate group, a trimethylsilyl group, an optionally substituted linear or branched alkyl group, an optionally substituted cycloalkyl group, an optionally substituted linear or branched alkenyl group, an optionally substituted cycloalkenyl group, an optionally substituted linear or branched alkynyl group, an optionally substituted linear or branched alkyloxy group, an optionally substituted phenyloxy group, an optionally substituted phenyl group, an optionally substituted naphthyl group, an optionally substituted 5- or 6-membered aromatic heterocyclic group, -SOₘR³ wherein R³ is an optionally substituted linear or branched alkyl group, an optionally substituted cycloalkyl group, an optionally substituted linear or branched alkenyl group, an optionally substituted cycloalkenyl group, an optionally substituted linear or branched alkynyl group, an optionally substituted aryl group, an optionally substituted pyridyl group, a - NR⁴R⁵ group wherein R⁴ and R⁵ are each a linear or branched alkyl group, and m is an integer of 0 to 2, or a 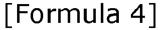 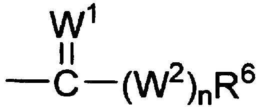 group wherein W¹ is an oxygen atom or a sulfur atom, W² is an oxygen atom, a sulfur atom, or -NH-, n is an integer of 0 or 1, and R⁶ is an optionally substituted linear or branched alkyl group or an optionally substituted phenyl group.

Further, according to another preferable embodiment of the present disclosure, R¹ and R² are each independently a hydrogen atom, a halogen atom, a nitro group, a cyano group,
an alkyl group optionally substituted with a halogen atom,
an alkyl group substituted with an alkyloxy group optionally substituted with a halogen atom,
an alkyl group substituted with a phenyl group,
an alkyl group substituted with a phenyl group substituted with an alkyl group, a halogen atom, or a hydroxyl group,
an alkenyl group optionally substituted with a halogen atom,
an alkyloxy group optionally substituted with a halogen atom,
a phenyl group optionally substituted with a halogen atom,
a phenyl group substituted with an alkyl group optionally substituted with a halogen atom,
a phenyl group substituted with an alkyloxy group optionally substituted with a halogen atom,
a thienyl group optionally substituted with a halogen atom,
a pyridyl group,
a furyl group,
a -S(O)ₘR³ group wherein R³ is an alkyl group optionally substituted with a phenyl group, a phenyl group optionally substituted with a halogen atom, a pyridyl group optionally substituted an alkyl group substituted with a halogen atom, an alkenyl group, or a -NR⁴R⁵ group wherein R⁴ and R⁵ are each an alkyl group, and m is an integer of 0 to 2, or
-C(=O)-(NH)ₙR⁶ wherein R⁶ is an alkyl group optionally substituted with a halogen atom or a phenyl group optionally substituted with a halogen atom, and n is an integer of 0 or 1.

Further, according to another preferable embodiment of the present disclosure, R¹ is an unsubstituted alkyl group, an alkyl group substituted with a halogen atom, an alkyl group substituted with a phenyl group optionally substituted with a halogen atom, an alkenyl group optionally substituted with a halogen atom, an alkylthio group, an unsubstituted phenyl group, a phenyl group substituted with a halogen atom, a phenyl group substituted with an alkyl group optionally substituted with a halogen atom, or a phenyl group substituted with an alkyloxy group optionally substituted with a halogen atom, and R² is a halogen atom.

Further, according to another preferable embodiment of the present disclosure, R¹ is a methyl group, an ethyl group, a n-propyl group, an isopropyl group, a n-butyl group, an isobutyl group, a n-pentyl group, a 3-chloro-n-propyl group, a 4-chloro-n-butyl group, an allyl group, an ethylthio group, a phenyl group, a 2-chlorophenyl group, a 2-fluorophenyl group, a 2-methylphenyl group, a 3-methylphenyl group, a 4-methylphenyl group, a 2-chloro-4-methylphenyl group, a 3-chloro-4-methylphenyl group, a 4-chloro-3-methylphenyl group, a benzyl group, or a 2-fluorobenzyl group, and R² is a chlorine atom or a bromine atom.

Further, according to a more preferable embodiment of the present disclosure, R¹ and R² are each independently a hydrogen atom, a halogen atom, or an optionally substituted phenyl group.

Further, according to a more preferable embodiment of the present disclosure, R¹ and R² are each independently a hydrogen atom or a halogen atom, or a phenyl group optionally substituted with at least one group of an alkyl group and a halogen atom.

Further, according to a more preferable embodiment of the present disclosure, R¹ and R² are each independently a hydrogen atom or a halogen atom, or a phenyl group optionally substituted with at least one group of an alkyl group having 1 to 6 carbon atoms and a halogen atom.

Further, according to an even more preferable embodiment of the present disclosure, R¹ and R² are different groups from each other.

Further, according to a more preferable embodiment of the present disclosure, X is a hydrogen atom, a hydroxyl group, or -OY, preferably a hydrogen atom or a hydroxyl group, and more preferably a hydrogen atom.

According to a more preferable embodiment of the present disclosure, Y is a group represented by the formula: 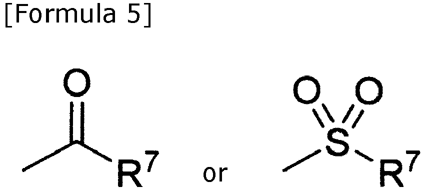 wherein R⁷ is an optionally substituted linear or branched alkyl group, an optionally substituted cycloalkyl group, an optionally substituted phenyl group, an optionally substituted thienyl group, an optionally substituted furyl group, or a -NR⁸R⁹ group wherein R⁸ and R⁹ are each independently a hydrogen atom, an optionally substituted alkyl group, or an optionally substituted alkenyl group, or together with a nitrogen atom adjacent to these, form a 5- to 7-membered saturated heterocycle, unless R⁸ and R⁹ are hydrogen atoms at the same time.

Further, according to another preferable embodiment of the present disclosure, R⁷ is an optionally substituted linear or branched alkyl group, an optionally substituted linear or branched alkyloxy group, an optionally substituted cycloalkyl group, an optionally substituted phenyl group, an optionally substituted phenyloxy group, a -NR⁸R⁹ group wherein R⁸ and R⁹ are each independently a hydrogen atom, an optionally substituted alkyl group, or an optionally substituted alkenyl group, or together with a nitrogen atom adjacent to these, form a 5- to 7-membered saturated heterocycle, unless R⁸ and R⁹ are hydrogen atoms at the same time, or a -CR¹⁰R¹¹R¹² group wherein R¹⁰, R¹¹, and R¹² are each independently an optionally substituted alkyl group or an optionally substituted phenyl group.

Further, according to another preferable embodiment of the present disclosure, R⁷ is an optionally substituted alkyl group, an optionally substituted phenyl group, an optionally substituted alkyloxy group, or an optionally substituted phenyloxy group, or a - CR¹⁰R¹¹R¹² group wherein R¹⁰, R¹¹, and R¹² are each independently an optionally substituted alkyl group or an optionally substituted phenyl group.

Further, according to a particularly preferable embodiment of the present disclosure, the compound represented by formula (I) or (II) is selected from 5-chloro-4-(4-methylphenyl)-1H-imidazole-2-carbonitrile, 4-(4-methylphenyl)-1H-imidazole-2-carbonitrile, 5-chloro-4-(3-methylphenyl)-1H-imidazole-2-carbonitrile, 5-chloro-4-(2-methylphenyl)-1H-imidazole-2-carbonitrile, 5-chloro-4-(2-chloro-4-methylphenyl)-1H-imidazole-2-carbonitrile, 5-chloro-4-(3-chloro-4-methylphenyl)-1H-imidazole-2-carbonitrile, 5-chloro-4-(4-chloro-3-methylphenyl)-1H-imidazole-2-carbonitrile, 4-(2-chloro-4-methylphenyl)-1H-imidazole-2-carbonitrile, 5-chloro-1-hydroxy-4-(4-methylphenyl)-1H-imidazole-2-carbonitrile, and 4-chloro-1-hydroxy-5-(4-methylphenyl)-1H-imidazole-2-carbonitrile.

The compound represented by formula (I) or (II) or a salt thereof can be produced by the methods described in Japanese Patent Laid-Open No. H8-283243, Japanese Patent Laid-Open No. 8-225539, and the like.

The compounds of the present disclosure may be used singly or in combinations of two or more.

According to a preferable embodiment, the compound of the present disclosure may be in the form of a salt. The salt is preferably a pharmaceutically or cosmetically acceptable salt. The pharmaceutically or cosmetically acceptable salt refers to a salt that can be used as a medicament. When the compound represented by formula (I) or (II) has an acidic group or a basic group, the compound can be formed into a basic salt or an acidic salt by reacting the compound with a base or an acid.

Examples of the acidic salt include an inorganic acid salt such as a hydrochloride, a hydrobromide, a sulfate, a nitrate, or a phosphate, and an organic acid salt such as an acetate, a propionate, a tartrate, a fumarate, a maleate, a malate, a citrate, a methanesulfonate, a benzenesulfonate, or a paratoluenesulfonate. Further, examples of the basic salt include an alkali metal salt such as a sodium salt or a potassium salt, and an alkaline earth metal salt such as a calcium salt or a magnesium salt.

The compound of the present disclosure or a salt thereof may absorb water to have adsorbed water attached or become a hydrate when left in the air or recrystallized, and the compound of the present disclosure or a salt thereof also encompasses such various hydrates, solvates, and crystal polymorphic compounds.

The compound of the present disclosure or a salt thereof may be used as an agent as it is, and if necessary, can be formulated by a conventional method by combining the same with a further additional component such as the above carrier, active component, pharmacological component, or cosmetic component.

According to a preferable embodiment of the present disclosure, in the above formulation, the content of the compound of the present disclosure is usually 0.00001 to 30% by mass, preferably 0.0001 to 10% by mass, and more preferably 0.001 to 0.05% by mass.

According to a preferable embodiment of the present disclosure, the carrier used in the above formulation is preferably a pharmaceutically or cosmetically acceptable carrier, and examples thereof include a carrier that can be usually used such as an excipient, a coating agent, a binding agent, a bulking agent, a disintegrating agent, a lubricant, a diluent, an osmotic pressure adjusting agent, a pH adjusting agent, a dispersing agent, an emulsifying agent, an antiseptic, a stabilizing agent, an antioxidant, a coloring agent, a UV absorber, a moisturizing agent, a thickening agent, an activity enhancer, a fragrance, a corrigent, and a flavoring agent.

Further, examples the further additional component that may be used include a moisturizing agent, an anti-inflammatory agent, a microbicidal agent, an antimicrobial agent, a UV protective agent, a cell activating agent, and a makeup component.

The formulation containing a compound of the present disclosure or a salt thereof can be provided as a pharmaceutical product, a quasi drug, or a cosmetic, and is preferably used as a medicament.

According to a preferable embodiment, the form of the formulation containing a compound of the present disclosure or a salt thereof can be any form such as a cream, a milky lotion, a lotion, a suspension, a gel, a powder, a pack, a sheet, a patch, a stick, or a cake, and is not particularly limited thereto.

Further, examples of a more specific form of the above formulation include, but are not particularly limited to, from the viewpoint of treatment of dermatitis or the like, an external medicament such as a lotion, a shampoo, a cream, a milky lotion, an ointment, or a patch, as well as a head or body cleanser such as a shampoo or a body shampoo, a rinse, a conditioner, a treatment, a hair pack, a hair tonic, a hair cream, and a head or body lotion, cream, milky lotion or the like.

The formulation of the present disclosure may be orally or parenterally administered. Examples of a dosage form for oral administration include a solid dosage form such as a tablet, a coated tablet, a granule, a powder, a capsule, and a liquid dosage form such as an elixir, a syrup, and a suspension. Examples of a dosage form for parenteral administration include an injection, an infusion, a topical, an external agent, a transdermal, a transmucosal, a transnasal, an enteral, an inhalation, a suppository, a bolus, and a patch. A preferable form of the formulation is a skin external agent.

The compound of the present disclosure or a salt thereof can immediately exert excellent antimicrobial activity against a fungus causing a human disease. Therefore, according to one embodiment, the formulation of the present disclosure is used for ameliorating a symptom or a disease caused by a fungus. Here, the "ameliorating" includes not only the treatment of an established pathological condition but also prophylaxis that is the prevention or the delay of a pathological condition that can be established in the future. Further, the ameliorating in the present disclosure preferably also includes improvement of a symptom or a condition of a skin disease, the prevention or the delay of worsening of a symptom or a condition of a skin disease, and the reversal, the prevention, or the delay of the progression of a symptom or a condition of a skin disease.

The fungus in the present disclosure is preferably a microbe belonging to the genus Candida, a microbe belonging to the genus Malassezia, a microbe belonging to the genus Trichophyton, a microbe belonging to the genus Microsporum, a microbe belonging to the genus Arthroderma, a microbe belonging to the genus Aspergillus, or a microbe belonging to the genus Cryptococcus, and more preferably a microbe belonging to the genus Candida, a microbe belonging to the genus Trichophyton, a microbe belonging to the genus Microsporum, a microbe belonging to the genus Arthroderma, a microbe belonging to the genus Aspergillus, or a microbe belonging to the genus Cryptococcus.

In the present disclosure, the microbe belonging to the genus Candida is a fungus belonging to the family Saccharomycetaceae, and examples thereof include Candida albicans and Candida glabrata.

In the present disclosure, the microbe belonging to the genus Malassezia is a fungus belonging to the family Malasseziaceae, and examples thereof include Malassezia furfur, Malassezia pachydermatis, Malassezia globosa, Malassezia obtusa, Malassezia restricta, Malassezia sympodialis, Malassezia slooffiae, Malassezia dermatis, Malassezia yamatoensis, Malassezia japonica, and Malassezia nana.

In the present disclosure, the microbe belonging to the genus Trichophyton is a fungus belonging to the family Arthrodermataceae, and examples thereof include Trichophyton rubrum and Trichophyton interdigitale.

In the present disclosure, the microbe belonging to the genus Microsporum is a fungus belonging to the family Arthrodermataceae, and examples thereof include Microsporum canis.

In the present disclosure, the microbe belonging to the genus Arthroderma is a fungus belonging to the family Arthrodermataceae, and examples thereof include Arthroderma vanbreuseqhemii.

In the present disclosure, the microbe belonging to the genus Aspergillus is a fungus belonging to the family Trichocomaceae, and examples thereof include Aspergillus fumigatus and Aspergillus niger.

In the present disclosure, the microbe belonging to the genus Cryptococcus is a fungus belonging to the family Tremellaceae, and examples thereof include Cryptococcus neoformans.

According to a preferable embodiment, the symptom or the disease caused by a fungus is a skin disease. Examples of the skin disease include tinea versicolor, psoriasis vulgaris, dermatophytosis, candidiasis, cutaneous malasseziosis, aspergillosis, cryptococcosis, atopic dermatitis, or otitis externa, and preferable examples thereof include dermatophytosis, candidiasis, aspergillosis, or cryptococcosis.

The administration target of the compound of the present disclosure or a salt thereof is preferably a human. The administration site of the compound of the present disclosure or a salt thereof is not particularly limited, and may be a tissue, an organ, or a cell of skin or the like in which the target fungus is present.

The compound of the present disclosure or a salt thereof can be administered in an effective amount for producing antifungal activity to a subject in need thereof to suppress the proliferation of, or sterilize, a fungus. Therefore, according to another embodiment of the present disclosure, a method for suppressing the proliferation of, or sterilizing, a fungus, including administering an effective amount of a compound represented by formula (I) or (II) to a subject in need thereof is provided. According to another preferable embodiment, the fungus is preferably a microbe belonging to the genus Candida, a microbe belonging to the genus Malassezia, a microbe belonging to the genus Trichophyton, a microbe belonging to the genus Microsporum, a microbe belonging to the genus Arthroderma, a microbe belonging to the genus Aspergillus, or a microbe belonging to the genus Cryptococcus, and more preferably a microbe belonging to the genus Candida, a microbe belonging to the genus Trichophyton, a microbe belonging to the genus Microsporum, a microbe belonging to the genus Arthroderma, a microbe belonging to the genus Aspergillus, or a microbe belonging to the genus Cryptococcus. Further, according to another preferable embodiment of the present disclosure, a method for ameliorating a symptom or a disease caused by a fungus, including administering an effective amount of a compound represented by formula (I) or (II) to a human subject in need thereof is provided.

The method of the present disclosure is preferably a therapeutic method and may be a non-therapeutic method. Specifically, the method of the present disclosure may be used for a cosmetic purpose or non-therapeutically used for a health promotion purpose. According to one embodiment, in a preferable embodiment of the present disclosure, the method does not include a medical practice, that is, a therapeutic treatment practice to an individual.

The effective amount of the compound of the present disclosure or a salt thereof varies depending on the animal species, sex, age, body weight, condition, and other factors of the administration target, and is an amount that reduces the proliferation of the target microbe to 50% or less, preferably 40% or less, more preferably 30% or less, further preferably 20% or less, and further more preferably 10% or less of that of the control.

In the above administration, the dose, administration route, and administration interval of the compound of the present disclosure vary depending on the animal species, sex, age, body weight, condition, and other factors of the administration target and thus cannot be unconditionally specified. For example, for the dosage of the compound of the present disclosure when topically administered to an affected area of a human, the daily dose is usually 0.005 to 350 mg/day, and the preferable weekly dose is 0.01 to 175 mg/week, per adult human weighing 60 kg.

Further, according to another embodiment of the present disclosure, use of a compound represented by formula (I) or (II) or a salt thereof in the production of an antifungal agent for a human is provided. Further, according to another preferable embodiment of the present disclosure, use of a compound represented by formula (I) or (II) or a salt thereof in the production of a formulation for ameliorating a symptom or a disease caused by a microbe belonging to the genus Malassezia is provided. According to another preferable embodiment, the fungus is a microbe belonging to the genus Candida, a microbe belonging to the genus Malassezia, a microbe belonging to the genus Trichophyton, a microbe belonging to the genus Microsporum, a microbe belonging to the genus Arthroderma, a microbe belonging to the genus Aspergillus, or a microbe belonging to the genus Cryptococcus. Further, according to another preferable embodiment of the present disclosure, the above symptom or disease is a skin disease. Further, according to another preferable embodiment of the present disclosure, the above symptom or disease is tinea versicolor, psoriasis vulgaris, dermatophytosis, candidiasis, cutaneous malasseziosis, aspergillosis, cryptococcosis, atopic dermatitis, or otitis externa. Further, according to another preferable embodiment of the present disclosure, the above antifungal agent is a medicament. Further, according to another preferable embodiment of the present disclosure, the above use is cosmetic or non-therapeutic use.

Further, according to another embodiment of the present disclosure, a compound represented by formula (I) or (II) or a salt thereof for use as an antifungal agent for a human is provided. Further, according to another preferable embodiment of the present disclosure, a compound represented by formula (I) or (II) or a salt thereof for ameliorating a symptom or a disease caused by a microbe belonging to the genus Malassezia is provided. According to another preferable embodiment, the fungus is a microbe belonging to the genus Candida, a microbe belonging to the genus Malassezia, a microbe belonging to the genus Trichophyton, a microbe belonging to the genus Microsporum, a microbe belonging to the genus Arthroderma, a microbe belonging to the genus Aspergillus, or a microbe belonging to the genus Cryptococcus. Further, according to another preferable embodiment of the present disclosure, the above symptom or disease is a skin disease. Further, according to another preferable embodiment of the present disclosure, the above symptom or disease is tinea versicolor, psoriasis vulgaris, dermatophytosis, candidiasis, cutaneous malasseziosis, aspergillosis, cryptococcosis, atopic dermatitis, or otitis externa. Further, according to another preferable embodiment of the present disclosure, the above antifungal agent is a medicament.

In addition, according to one embodiment of the present disclosure, the following are provided.
[1] An antifungal agent for a human comprising a compound represented by formula (I) or (II) or a salt thereof 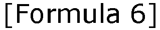 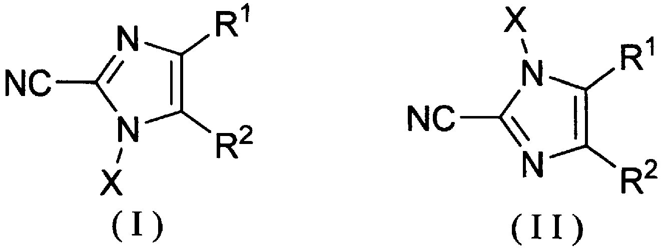 wherein
   R¹ and R² are each independently
   a hydrogen atom,
   a halogen atom,
   a hydroxyl group,
   a nitro group,
   a cyano group,
   a thiocyanate group,
   a trimethylsilyl group,
   an optionally substituted alkyl group,
   an optionally substituted alkenyl group,
   an optionally substituted alkynyl group,
   an optionally substituted alkyloxy group,
   an optionally substituted alkenyloxy group,
   an optionally substituted alkynyloxy group,
   an optionally substituted aryl group,
   an optionally substituted aryloxy group,
   an optionally substituted 5- or 6-membered aromatic heterocyclic group,

      -SOₘR³
   wherein R³ is an optionally substituted alkyl group, an optionally substituted alkenyl group, an optionally substituted alkynyl group, an optionally substituted aryl group, an optionally substituted 5- or 6-membered aromatic heterocyclic group, or a -NR⁴R⁵ group wherein R⁴ and R⁵ are each an optionally substituted alkyl group, and m is an integer of 0 to 2, or
   a group represented by 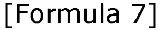 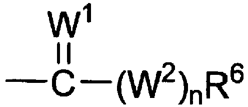
   wherein
   W¹ is an oxygen atom or a sulfur atom,
   W² is an oxygen atom, a sulfur atom, or -NH-
   n is an integer of 0 or 1,
   R⁶ is an optionally substituted alkyl group or an optionally substituted aryl group;
   X is a hydrogen atom, a hydroxyl group, or -OY; and
   Y is a formula: 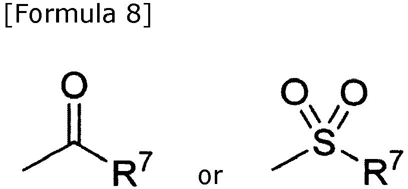
   wherein
   R⁷ is an optionally substituted alkyl group,
   an optionally substituted alkyloxy group,
   an optionally substituted alkenyl group,
   an optionally substituted alkenyloxy group,
   an optionally substituted aryl group,
   an optionally substituted aryloxy group,
   an optionally substituted 5- or 6-membered aromatic heterocyclic group,
   a -NR⁸R⁹ group wherein R⁸ and R⁹ are each independently a hydrogen atom, an optionally substituted alkyl group, or an optionally substituted alkenyl group, or together with a nitrogen atom adjacent to these, form a 5- to 7-membered saturated heterocycle, unless R⁸ and R⁹ are hydrogen atoms at the same time, or
   a -CR¹⁰R¹¹R¹² group wherein R¹⁰, R¹¹, and R¹² are each independently an optionally substituted alkyl group, an optionally substituted alkenyl group, or an optionally substituted aryl group.
[2] The antifungal agent according to [1], wherein R¹ and R² are each independently a hydrogen atom, a halogen atom, or an optionally substituted phenyl group.
[3] The antifungal agent according to [1] or [2], wherein R¹ and R² are each independently a hydrogen atom or a halogen atom, or a phenyl group optionally substituted with at least one group of an alkyl group and a halogen atom.
[4] The antifungal agent according to any one of [1] to [3], wherein R¹ and R² are each independently a hydrogen atom or a halogen atom, or a phenyl group optionally substituted with at least one group of an alkyl group having 1 to 6 carbon atoms and a halogen atom.
[5] The antifungal agent according to any one of [1] to [4], wherein R¹ and R² are different groups from each other.
[6] The antifungal agent according to any one of [1] to [5], wherein X is a hydrogen atom, a hydroxyl group, or -OY; and Y is a group represented by a formula: 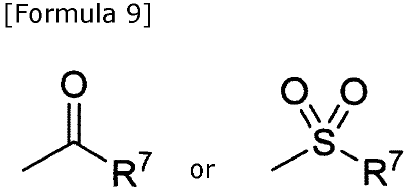 wherein R⁷ is an optionally substituted alkyl group, an optionally substituted phenyl group, an optionally substituted alkyloxy group, an optionally substituted phenyloxy group, or a -CR¹⁰R¹¹R¹² group wherein R¹⁰, R¹¹, and R¹² are each independently an optionally substituted alkyl group or an optionally substituted phenyl group.
[7] The antifungal agent according to any one of [1] to [6], wherein X is a hydrogen atom or a hydroxyl group.
[8] The antifungal agent according to any one of [1] to [7], wherein the compound represented by formula (I) or (II) is selected from 5-chloro-4-(4-methylphenyl)-1H-imidazole-2-carbonitrile, 4-(4-methylphenyl)-1H-imidazole-2-carbonitrile, 5-chloro-4-(3-methylphenyl)-1H-imidazole-2-carbonitrile, 5-chloro-4-(2-methylphenyl)-1H-imidazole-2-carbonitrile, 5-chloro-4-(2-chloro-4-methylphenyl)-1H-imidazole-2-carbonitrile, 5-chloro-4-(3-chloro-4-methylphenyl)-1H-imidazole-2-carbonitrile, 5-chloro-4-(4-chloro-3-methylphenyl)-1H-imidazole-2-carbonitrile, 4-(2-chloro-4-methylphenyl)-1H-imidazole-2-carbonitrile, 5-chloro-1-hydroxy-4-(4-methylphenyl)-1H-imidazole-2-carbonitrile, and 4-chloro-1-hydroxy-5-(4-methylphenyl)-1H-imidazole-2-carbonitrile.
[9] The antifungal agent according to any one of [1] to [8], wherein the antifungal agent is a medicament.
[10] The antifungal agent according to any one of [1] to [9], wherein the fungus is at least one selected from a microbe belonging to the genus Candida, a microbe belonging to the genus Malassezia, a microbe belonging to the genus Trichophyton, a microbe belonging to the genus Microsporum, a microbe belonging to the genus Arthroderma, a microbe belonging to the genus Aspergillus, and a microbe belonging to the genus Cryptococcus.
[11] The antifungal agent according to any one of [1] to [10], wherein the fungus is at least one selected from a microbe belonging to the genus Candida, a microbe belonging to the genus Trichophyton, a microbe belonging to the genus Microsporum, a microbe belonging to the genus Arthroderma, a microbe belonging to the genus Aspergillus, and a microbe belonging to the genus Cryptococcus.
[12] The antifungal agent according to any one of [1] to [11] for ameliorating a symptom or a disease caused by the fungus.
[13] The antifungal agent according to [12], wherein the symptom or disease is a skin disease.
[14] The antifungal agent according to [12] or [13], wherein the symptom or disease is tinea versicolor, psoriasis vulgaris, dermatophytosis, candidiasis, cutaneous malasseziosis, aspergillosis, cryptococcosis, atopic dermatitis, or otitis externa.
[15] The antifungal agent according to any one of [12] to [14], wherein the symptom or disease is cutaneous malasseziosis, dermatophytosis, candidiasis, aspergillosis, or cryptococcosis.

### Examples

Next, test examples according to the present invention will be described, but these do not limit the present invention.

### Production Example 1: 5-Chloro-4-(4-methylphenyl)-1H-imidazole-2-carbonitrile (compound No. 1)

5-Chloro-4-(4-methylphenyl)-1H-imidazole-2-carbonitrile (compound No. 1) was produced according to the method described in Examples 1 to 5 of Japanese Patent Laid-Open No. H8-225539 (CAS number: 120118-14-1).

### Production Example 2: 4-(4-Methylphenyl)-1H-imidazole-2-carbonitrile (compound No. 2)

Compound No. 2 was produced according to the same method as described in Japanese Patent Laid-Open No. H8-225539 except that a raw material compound corresponding to the production target was used (CAS number: 120118-10-7).

MS: 184.2 [M+H]⁺

### Production Example 3: 5-Chloro-4-(3-methylphenyl)-1H-imidazole-2-carbonitrile (compound No. 3)

Compound No. 3 was produced according to the same method as described in Japanese Patent Laid-Open No. H8-225539 except that a raw material compound corresponding to the production target was used (CAS number: 120118-18-5).

MS: 218.2 [M+H]⁺

### Production Example 4: 5-Chloro-4-(2-methylphenyl)-1H-imidazole-2-carbonitrile (compound No. 4)

Compound No. 4 was produced according to the same method as described in Japanese Patent Laid-Open No. H8-225539 except that a raw material compound corresponding to the production target was used.

### ¹H-NMR (DMSO-d6): δ 7.45-7.31 (m, 4H), 3.45-3.28 (br, 1H), 2.23 (s, 3H)

MS: 218.1 [M+H]⁺

### Production Example 5: 5-Chloro-4-(2-chloro-4-methylphenyl)-1H-imidazole-2-carbonitrile (compound No. 5)

Compound No. 5 was produced according to the same method as described in Japanese Patent Laid-Open No. H8-225539 except that a raw material compound corresponding to the production target was used (CAS number: 2167064-69-7).

MS: 252.2 [M+H]⁺

### Production Example 6: 5-Chloro-4-(3-chloro-4-methylphenyl)-1H-imidazole-2-carbonitrile (compound No. 6)

Compound No. 6 was produced according to the same method as described in Japanese Patent Laid-Open No. H8-225539 except that a raw material compound corresponding to the production target was used (CAS number: 120118-82-3).

MS: 252.1 [M+H]⁺

### Production Example 7: 5-Chloro-4-(4-chloro-3-methylphenyl)-1H-imidazole-2-carbonitrile (compound No. 7)

Compound No. 7 was produced according to the same method as described in Japanese Patent Laid-Open No. H8-225539 except that a raw material compound corresponding to the production target was used.
¹H-NMR (DMSO-d6): δ 7.73 (s, 1H), 7.62-7.58 (m, 2H), 3.80-3.00 (br, 1H), 2.40 (s, 3H)
MS: 251.0 [M+H]⁺

### Production Example 8: 4-(2-chloro-4-methylphenyl)-1H-imidazole-2-carbonitrile (compound No. 8)

Compound No. 8 was produced according to the same method as described in Japanese Patent Laid-Open No. H8-225539 except that a raw material compound corresponding to the production target was used.
¹H-NMR (DMSO-d6): δ 8.10-7.93 (br, 1H), 7.93-7.77 (br, 1H), 7.38 (s, 1H), 7.24 (d, 1H, J=8.0 Hz), 3.32 (s, 1H), 2.34 (s, 3H)
MS: 218.1 [M+H]⁺

### Production Example 9: 5-Chloro-1-hydroxy-4-(4-methylphenyl)-1H-imidazole-2-carbonitrile (compound No. 9)

Compound No. 9 was produced according to the same method as described in Japanese Patent Laid-Open No. H8-225539 except that a raw material compound corresponding to the production target was used.
¹H-NMR (DMSO-d6): δ 7.72 (d, 2H, J=8.0 Hz), 7.26 (d, 2H, J=8.0 Hz), 3.45-3.15 (br, 1H), 2.31 (s, 3H)
MS: 234.1 [M+H]⁺

### Production Example 10: 4-Chloro-1-hydroxy-5-(4-methylphenyl)-1H-imidazole-2-carbonitrile (compound No. 10)

Compound No. 10 was produced according to the same method as described in Japanese Patent Laid-Open No. H8-225539 except that a raw material compound corresponding to the production target was used (CAS number: 177762-70-8).

MS: 234.1 [M+H]⁺

### Test Example 1-1: Antimicrobial activity test

Test compound: Compound No. 1
Test microbial strain: Candida albicans (TIMM1768 strain)
Test method: The Candida microbe cultured in an YPD liquid medium at 37°C for 20 hours was recovered by centrifugation (3000 rpm, 5 minutes), washed twice with physiological saline, and then suspended in physiological saline, and a microbial liquid was prepared in such a way as to provide 2 × 10³ cells/mL. 100 µL of the microbial liquid was applied to a Sabouraud medium to which the test compound dissolved in DMSO was added to have a predetermined concentration. After culturing at 37°C for 48 hours, the presence or absence of colony formation was investigated.

As a result, compound No. 1 completely inhibited colony formation at 50 ppm.

### Test Example 1-2: Antimicrobial activity test

Test compound: Compound No. 1
Test microbial strain: Candida glabrata (TIMM6054 strain)
Test method: The Candida microbe cultured in an YPD liquid medium at 37°C for 20 hours was recovered by centrifugation (3000 rpm, 5 minutes), washed twice with physiological saline, and then suspended in physiological saline, and a microbial liquid was prepared in such a way as to provide 2 × 10³ cells/mL. 100 µL of the microbial liquid was applied to an YPD medium to which the test compound dissolved in DMSO was added to have a predetermined concentration. After culturing at 37°C for 48 hours, the presence or absence of colony formation was investigated.

As a result, compound No. 1 completely suppressed colony formation at 200 ppm.

### Test Example 1-3: Antimicrobial activity test

Test compound: Compound No. 1
Test microbial strain: Cryptococcus neoformans (TIMM1316 strain) Test method: The Cryptococcus microbe cultured in an YPD liquid medium at 37°C for 20 hours was recovered by centrifugation (3000 rpm, 5 minutes), washed twice with physiological saline, and then suspended in physiological saline, and a microbial liquid was prepared in such a way as to provide 2 × 10³ cells/mL. 100 µL of the microbial liquid was applied to a Sabouraud medium to which the test compound dissolved in DMSO was added to have a predetermined concentration. After culturing at 37°C for 48 hours, the presence or absence of colony formation was investigated.

As a result, compound No. 1 completely suppressed colony formation at 20 ppm.

### Test Example 1-4: Antimicrobial activity test

Test compound: Compound No. 1
Test microbial strain: Malassezia nana (CBS95574)

The Malassezia microbe cultured at 32°C for 20 hours in a Sabouraud liquid medium containing Tween80 (polyoxyethylene sorbitan monooleate: manufactured by Kanto Chemical Co., Inc.) (0.05% (v/v)) was recovered by centrifugation (3000 rpm, 5 minutes), washed twice with physiological saline, and then suspended in 10 mL of physiological saline, the resulting suspension was allowed to stand for 15 minutes, and then the supernatant was recovered. Using this supernatant and physiological saline, a microbial liquid of McFarland No. 0.5 was prepared, compound No. 1 dissolved in DMSO was added in such a way as to provide 100 ppm or 200 ppm, the resulting microbial liquid was stirred and allowed to stand at 30°C for 5 minutes, then 100 µL of the microbial liquid was suspended in 10 mL physiological saline, and the Malassezia microbe was recovered by centrifugation (3,000 rpm, 5 minutes), washed once with physiological saline, and then resuspended in 500 µL of physiological saline. 100 µL of the microbial liquid resulting from the resuspension was applied to a Sabouraud medium containing Tween80 (0.05% (v/v)) and subjected to culturing at 30°C for 5 days, then the number of colonies formed there (number of colonies formed in the inventive plot) and the number of colonies in a solvent to which the test compound was not added (the number of colonies formed in the non-agent treated plot) were measured, and the colony formation inhibition rate (%) was determined according to the following expression.

Colony formation inhibition rate (%) = [(number of colonies formed in non-agent treated plot - number of colonies formed in inventive plot)/number of colonies formed in non-agent treated plot] × 100

As a result, the colony formation inhibition rate of compound No. 1 was 98.5% at 100 ppm and 99.9% at 200 ppm.

### Test Example 1-5: Antimicrobial activity test

Test compound: Compound No. 1
Test microbial strain: Malassezia pachydermatis (TIMM10024)

The Malassezia microbe cultured at 32°C for 20 hours in a Sabouraud liquid medium containing Tween80 (polyoxyethylene sorbitan monooleate: manufactured by Kanto Chemical Co., Inc.) (0.05% (v/v)) was recovered by centrifugation (3000 rpm, 5 minutes), washed twice with physiological saline, and then suspended in 10 mL of physiological saline, the resulting suspension was allowed to stand for 15 minutes, and then the supernatant was recovered. Using this supernatant and physiological saline, a microbial liquid of McFarland No. 0.5 was prepared, compound No. 1 dissolved in DMSO was added in such a way as to provide 100 ppm or 200 ppm, the resulting microbial liquid was stirred and allowed to stand at 30°C for 5 minutes, then 100 µL of the microbial liquid was suspended in 10 mL physiological saline, and the Malassezia microbe was recovered by centrifugation (3,000 rpm, 5 minutes), washed once with physiological saline, and then resuspended in 500 µL of physiological saline. 100 µL of the microbial liquid resulting from the resuspension was applied to a Sabouraud medium containing Tween80 (0.05% (v/v)) and subjected to culturing at 30°C for 5 days, then the number of colonies formed there (number of colonies formed in the inventive plot) and the number of colonies in a solvent to which the test compound was not added (the number of colonies formed in the non-agent treated plot) were measured, and the colony formation inhibition rate (%) was determined in the same manner as in Test Example 1-4.

As a result, the colony formation inhibition rate of compound No. 1 was 98.1% at 100 ppm and 100% at 200 ppm.

### Test Example 1-6: Antimicrobial activity test

Test compound: Compound No. 1
Test microbial strains: Aspergillus fumigatus (TIMM0063 strain), and A. niger (TIMM4968 strain)
Test method: The Aspergillus microbe was cultured in a PDA medium at 27°C for 3 days, then spores were collected in physiological saline containing Tween80 (polyoxyethylene sorbitan monooleate: manufactured by Kanto Chemical Co., Inc.) (0.05% (v/v)), and this was filtered through a cell strainer (ϕ40 µm). The spores were recovered from the filtrate by centrifugation (3500 rpm, 5 minutes) and washed twice with physiological saline, and then a spore suspension was prepared (1 × 10⁷ spores/mL). 10 µL of the spore suspension was inoculated into a Sabouraud medium to which compound No. 1 dissolved in DMSO was added to have a predetermined concentration. After culturing at 28°C for 48 hours, the presence or absence of microbial growth was investigated, and the minimum inhibitory concentration was determined.

As a result, the minimum inhibitory concentration of compound No. 1 for each of the two microbial strains was 100 ppm.

### Test Example 1-7: Antimicrobial activity test

Test compound: Compound No. 1
Test microbial strains: Trichophyton rubrum (CBS118892 strain), T. interdegitale (TIMM20065 strain), Microsporum canis (TIMM20080 strain), and Arthroderma vanbreuseqhemii (TIMM2789)

Each microbial strain was cultured in a Sabouraud medium containing 500 µg/mL of cycloheximide and 50 µg/mL of chloramphenicol at 28°C for 8 days, then spores were collected in physiological saline containing Tween80 (polyoxyethylene sorbitan monooleate: manufactured by Kanto Chemical Co., Inc.) (0.05% (v/v)), and this was filtered through a cell strainer (ϕ40 µm). The spores were recovered from the filtrate by centrifugation (3500 rpm, 5 minutes) and washed twice with physiological saline, and then a spore suspension was prepared (1 × 10⁷ spores/mL). 10 µL of the spore suspension was inoculated into a Sabouraud medium to which compound No. 1 dissolved in DMSO was added to have a predetermined concentration. After culturing at 28°C for 72 hours, the presence or absence of microbial growth was investigated, and the minimum inhibitory concentration was determined.

As a result, the minimum inhibitory concentration of compound No. 1 for each of the four microbial strains was 10 ppm.

### Test Example 2

Test compound: Compound No. 6
Test microbial strains: Candida albicans (NBRC1594 strain), Candida glabrata (JCM3761 strain), Cryptococcus neoformans (JCM3685 strain), Aspergillus fumigatus (JCM10253), Aspergillus niger (ATCC6725), Trichophyton rubrum (ATCCMYA4607), Trichophyton interdegitale (ATCC9533), Microsporum canis (NBRC7863), and Arthroderma vanbreuseqhemii (JCM1891)

The Candida microbes and the Cryptococcus microbe were cultured at 30°C for 72 hours and the other microbes were cultured at 25°C for 120 hours in Sabouraud media, and then suspended in 5 mL of PBS (containing 8 g of sodium chloride, 2.9 g of disodium hydrogen phosphate, 0.2 g of potassium chloride, and 0.2 g of potassium dihydrogen phosphate in 1000 mL) containing Tween80 (polyoxyethylene sorbitan monooleate: Kanto Chemical Co., Inc.) (1% (V/V))), and the resulting suspension was filtered through a cell strainer (ϕ40 µm). Spores were recovered from the filtrate by centrifugation (3500 rpm, 5 minutes) and washed twice with physiological saline, and then a spore suspension was prepared (1 × 10⁶ spores/mL). 50 µL of the spore suspension was inoculated into a Sabouraud medium to which compound No. 6 dissolved in DMSO was added to have a predetermined concentration. The Candida microbes and the Cryptococcus microbe were cultured at 30°C for 72 hours and the other microbes were cultured at 25°C for 120 hours, then the presence or absence of colony growth was investigated, and the minimum inhibitory concentration was determined.

Results thereof are shown in Table 1.

### [Table 1]

**Table 1**

| Microbial species | Minimum inhibitory concentration |
|---|---|
| Candida albicans | 250 ppm |
| Candida glabrata | 250 ppm |
| Cryptococcus neoformans | 31.3 ppm |
| Aspergillus fumigatus | 62.5 ppm |
| Aspergillus niger | 62.5 ppm |
| Trichophyton rubrum | 31.3 ppm |
| Trichophyton interdegitale | 62.5 ppm |
| Microsporum canis | 125 ppm |
| Arthroderma vanbreuseghemii | 7.8 ppm |

### Test Example 3-1: Antifungal activity test

Test compound: Compound No. 6
Test microbial strains: Malassezia furfur (IFM 55951), Malassezia sympodialis (IFM 48588), and Malassezia globosa (IFM 51946)

The Malassezia microbe (Malassezia furfur, Malassezia sympodialis, or Malassezia globosa) cultured in a CHROMagarTM Malassezia/Candida Prepared medium (peptone, special enzyme substrate mixture, chloramphenicol, olive oil, agar, manufactured by Kanto Chemical Co., Inc.) for 5 to 7 days was suspended in physiological saline, and a suspension thereof was prepared in such a way as to have an absorbance of 0.025 at 600 nm. Thereto, the test compound dissolved in DMSO was added in such a way as to provide a final concentration of 200, 100, 50, or 25 ppm, and after 1 minute, 10 µL of the suspension and 10 mL of physiological saline were placed in a syringe and stirred, and the syringe was connected to a 37 mm Quality Monitor (manufactured by Pall Corporation) followed by filtration and washing twice with 10 mL of physiological saline. The membrane filter was removed from the Quality Monitor, placed on a CHROMagarTM Malassezia/Candida Prepared medium, and was subjected to culturing at 32°C for 72 hours, the presence or absence of colony growth was investigated, and the minimum fungicidal concentration (MFC100) was determined. Results thereof are shown in Table 2-1.

### [Table 2-1]

**Table 2-1**

| Microbial species | Minimum fungicidal concentration (MFC100) |
|---|---|
| Malassezia furfur | 50 ppm |
| Malassezia sympodialis | 50 ppm |
| Malassezia globosa | 50 ppm |

### Test Example 3-2: Antifungal activity test

Test compound: Compound No. 6
Test microbial strain: Malassezia restricta (IFM55992)

The Malassezia microbe (Malassezia restricta) cultured in a CHROMagarTM Malassezia/Candida Prepared medium (peptone, special enzyme substrate mixture, chloramphenicol, olive oil, agar, manufactured by Kanto Chemical Co., Inc.) for 5 to 7 days was suspended in physiological saline, and a suspension thereof was prepared in such a way as to have an absorbance of 0.025 at 600 nm. Thereto, the test compound dissolved in DMSO was added in such a way as to provide a final concentration of 200, 100, 50, or 25 ppm, and after 1 minute, 10 µL of the suspension and 10 mL of physiological saline were placed in a syringe and stirred, and the syringe was connected to a 37 mm Quality Monitor (manufactured by Pall Corporation) followed by filtration and washing twice with 10 mL of physiological saline. The membrane filter was removed from the Quality Monitor, placed on a Leeming & Notman agar Modified medium (MLNA medium) (peptone, glucose, yeast extract, dried bovine bile, glycerol, glycerin stearate, Tween60, olive oil, agar), and subjected to culturing at 32°C for 72 hours, the presence or absence of colony growth was investigated, and the minimum fungicidal concentration MFC100) was determined. Results thereof are shown in Table 2-2.

### [Table 2-2]

**Table 2-2**

| Microbial species | Minimum fungicidal concentration (MFC100) |
|---|---|
| Malassezia restricta | 200 ppm |

### Test Example 4: Antifungal activity test

Test compound: Compound No. 6
Test microbial strains: Trichophyton rubrum (ATCCMYA4607), and Microsporum canis (IFM63627)

The antifungal activity was studied by partially modifying the method of Test Example 2. Trichophyton rubrum was cultured in a Sabouraud medium and Microsporum canis was cultured in a 1/10 Sabouraud medium, at 32°C for 168 hours to form spores. For Trichophyton rubrum, hyphae were peeled off from the surface of the medium and recovered in a tube, physiological saline (Otsuka Normal Saline; containing 9 g of sodium chloride in 1000 mL) was added, these were wholly admixed using a disposable loop (type 1 (1 µL)) for dispersion, and a spore suspension containing the hyphae was recovered. For Microsporum canis, 10 mL of physiological saline was added directly onto the medium, and the surface was rubbed using a disposable loop to recover a spore suspension containing hyphae. The spore suspensions containing the hyphae of Trichophyton rubrum and Microsporum canis, respectively, were filtered through a cell strainer (ϕ40 µm). The spores were recovered from the filtrate by centrifugation (3500 rpm, 5 minutes) and washed twice with physiological saline, and then a spore suspension was prepared (1 × 10⁷ spores/mL). 10 µL of the spore suspension was inoculated into a Sabouraud medium to which the test compound dissolved in DMSO was added to have a predetermined concentration. Trichophyton rubrum, Microsporum canis was cultured at 32°C for 72 hours, then the presence or absence of colony growth was investigated, and the minimum inhibitory concentration (MIC100) was determined. Results thereof are shown in Table 3.

### [Table 3]

**Table 3**

| Microbial species | Minimum inhibitory concentration (MIC100) |
|---|---|
| Trichophyton rubrum | 5 ppm |
| Microsporum canis | 5 ppm |

### Test Example 5: Antifungal activity test

Test compound: Compound No. 6
Test microbial strains: Trichophyton rubrum (ATCCMYA4607), and Microsporum canis (IFM63627)

Trichophyton rubrum was cultured in a Sabouraud medium and Microsporum canis was cultured in a 1/10 Sabouraud medium, at 32°C for 168 hours to form spores. For Trichophyton rubrum, hyphae were peeled off from the surface of the medium and recovered in a tube, physiological saline (Otsuka Normal Saline; containing 9 g of sodium chloride in 1000 mL) was added, these were wholly admixed using a disposable loop (type 1 (1 µL)) for dispersion, and a spore suspension containing the hyphae was recovered. For Microsporum canis, 10 mL of physiological saline was added directly onto the medium, and the surface was rubbed using a disposable loop to recover a spore suspension containing hyphae. The spore suspensions containing the hyphae of Trichophyton rubrum and Microsporum canis, respectively, were filtered through a cell strainer (ϕ40 µm). The spores were recovered from the filtrate by centrifugation (3500 rpm, 5 minutes) and washed twice with physiological saline, and then a spore suspension was prepared (1 × 10⁷ spores/mL). 10 µL of the spore suspension was inoculated into 90 µL of physiological saline to which test compound dissolved in DMSO was added to have a predetermined concentration. Every time a predetermined time elapsed, 10 µL of a test liquid containing the spores was collected, the spores were recovered by centrifugation (3500 rpm, 5 minutes) and washed twice with physiological saline, and then a 10-µL spore suspension was prepared. The 10-µL spore suspension was spotted onto a Sabouraud medium and subjected to culturing at 32°C for 72 hours, the presence or absence of colony growth was investigated, and the minimum fungicidal concentration (MFC100) was determined. Results thereof are shown in Table 4.

### [Table 4]

**Table 4**

| Microbial species | Treatment time | Minimum fungicidal concentration (MFC100) |
|---|---|---|
| Trichophyton rubrum | 1 Hour | 50 ppm |
| | 48 Hours | 10 ppm |
| | 72 Hours | 5 ppm |
| Microsporum canis | 24 Hours | 50 ppm |
| | 96 Hours | 10 ppm |

## Claims

1. An antifungal agent for a human comprising a compound represented by formula (I) or (II) or a salt thereof 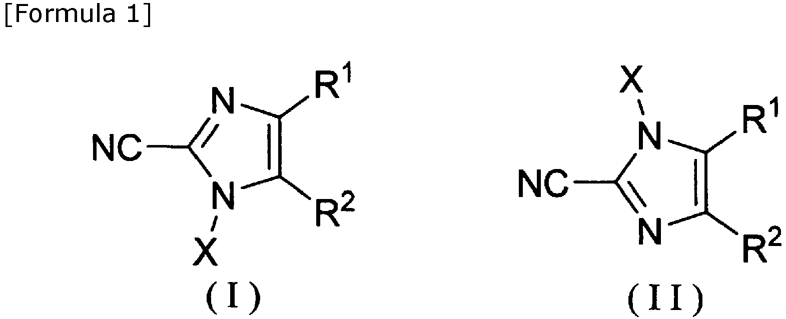 wherein
R¹ and R² are each independently
a hydrogen atom,
a halogen atom,
a hydroxyl group,
a nitro group,
a cyano group,
a thiocyanate group,
a trimethylsilyl group,
an optionally substituted alkyl group,
an optionally substituted alkenyl group,
an optionally substituted alkynyl group,
an optionally substituted alkyloxy group,
an optionally substituted alkenyloxy group,
an optionally substituted alkynyloxy group,
an optionally substituted aryl group,
an optionally substituted aryloxy group,
an optionally substituted 5- or 6-membered aromatic heterocyclic group,
-SOₘR³
wherein R³ is an optionally substituted alkyl group, an optionally substituted alkenyl group, an optionally substituted alkynyl group, an optionally substituted aryl group, an optionally substituted 5- or 6-membered aromatic heterocyclic group, or a -NR⁴R⁵ group wherein
R⁴ and R⁵ are each an optionally substituted alkyl group, and m is an integer of 0 to 2, or
a group represented by 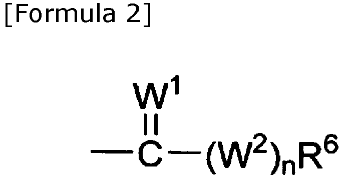
wherein
W¹ is an oxygen atom or a sulfur atom,
W² is an oxygen atom, a sulfur atom, or -NH-
n is an integer of 0 or 1,
R⁶ is an optionally substituted alkyl group or an optionally substituted aryl group;
X is a hydrogen atom, a hydroxyl group, or -OY; and
Y is a formula: 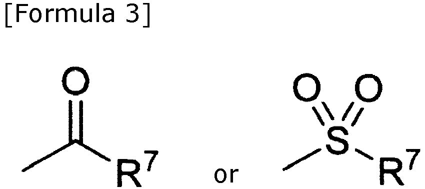
wherein
R⁷ is an optionally substituted alkyl group,
an optionally substituted alkyloxy group,
an optionally substituted alkenyl group,
an optionally substituted alkenyloxy group,
an optionally substituted aryl group,
an optionally substituted aryloxy group,
an optionally substituted 5- or 6-membered aromatic heterocyclic group,
a -NR⁸R⁹ group wherein R⁸ and R⁹ are each independently a hydrogen atom, an optionally substituted alkyl group, or an optionally substituted alkenyl group, or together with a nitrogen atom adjacent to these, form a 5- to 7-membered saturated heterocycle, unless R⁸ and R⁹ are hydrogen atoms at the same time, or
a -CR¹⁰R¹¹R¹² group wherein R¹⁰, R¹¹, and R¹² are each independently an optionally substituted alkyl group, an optionally substituted alkenyl group, or an optionally substituted aryl group.

2. The antifungal agent according to claim 1, wherein R¹ and R² are each independently a hydrogen atom, a halogen atom, or an optionally substituted phenyl group.

3. The antifungal agent according to claim 1 or 2, wherein R¹ and R² are each independently a hydrogen atom or a halogen atom, or a phenyl group optionally substituted with at least one group of an alkyl group and a halogen atom.

4. The antifungal agent according to any one of claims 1 to 3, wherein R¹ and R² are each independently a hydrogen atom or a halogen atom, or a phenyl group optionally substituted with at least one group of an alkyl group having 1 to 6 carbon atoms and a halogen atom.

5. The antifungal agent according to any one of claims 1 to 4, wherein R¹ and R² are different groups from each other.

6. The antifungal agent according to any one of claims 1 to 5, wherein
X is a hydrogen atom, a hydroxyl group, or -OY; and
Y is a group represented by a formula: 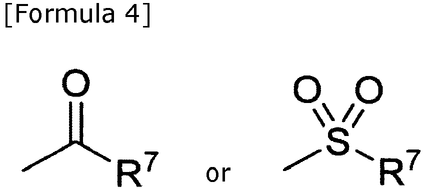
wherein R⁷ is an optionally substituted alkyl group, an optionally substituted phenyl group, an optionally substituted alkyloxy group, an optionally substituted phenyloxy group, or a -CR¹⁰R¹¹R¹² group wherein R¹⁰, R¹¹, and R¹² are each independently an optionally substituted alkyl group or an optionally substituted phenyl group.

7. The antifungal agent according to any one of claims 1 to 6, wherein X is a hydrogen atom or a hydroxyl group.

8. The antifungal agent according to any one of claims 1 to 7, wherein the compound represented by formula (I) or (II) is selected from 5-chloro-4-(4-methylphenyl)-1H-imidazole-2-carbonitrile, 4-(4-methylphenyl)-1H-imidazole-2-carbonitrile, 5-chloro-4-(3-methylphenyl)-1H-imidazole-2-carbonitrile, 5-chloro-4-(2-methylphenyl)-1H-imidazole-2-carbonitrile, 5-chloro-4-(2-chloro-4-methylphenyl)-1H-imidazole-2-carbonitrile, 5-chloro-4-(3-chloro-4-methylphenyl)-1H-imidazole-2-carbonitrile, 5-chloro-4-(4-chloro-3-methylphenyl)-1H-imidazole-2-carbonitrile, 4-(2-chloro-4-methylphenyl)-1H-imidazole-2-carbonitrile, 5-chloro-1-hydroxy-4-(4-methylphenyl)-1H-imidazole-2-carbonitrile, and 4-chloro-1-hydroxy-5-(4-methylphenyl)-1H-imidazole-2-carbonitrile.

9. The antifungal agent according to any one of claims 1 to 8, wherein the antifungal agent is a medicament.

10. The antifungal agent according to any one of claims 1 to 9, wherein the fungus is at least one selected from a microbe belonging to the genus Candida, a microbe belonging to the genus Malassezia, a microbe belonging to the genus Trichophyton, a microbe belonging to the genus Microsporum, a microbe belonging to the genus Arthroderma, a microbe belonging to the genus Aspergillus, and a microbe belonging to the genus Cryptococcus.

11. The antifungal agent according to any one of claims 1 to 10, wherein the fungus is at least one selected from a microbe belonging to the genus Candida, a microbe belonging to the genus Trichophyton, a microbe belonging to the genus Microsporum, a microbe belonging to the genus Arthroderma, a microbe belonging to the genus Aspergillus, and a microbe belonging to the genus Cryptococcus.

12. The antifungal agent according to any one of claims 1 to 11 for ameliorating a symptom or a disease caused by the fungus.

13. The antifungal agent according to claim 12, wherein the symptom or disease is a skin disease.

14. The antifungal agent according to claim 12 or 13, wherein the symptom or disease is tinea versicolor, psoriasis vulgaris, dermatophytosis, candidiasis, cutaneous malasseziosis, aspergillosis, cryptococcosis, atopic dermatitis, or otitis externa.

15. The antifungal agent according to any one of claims 12 to 14, wherein the symptom or disease is cutaneous malasseziosis, dermatophytosis, candidiasis, aspergillosis, or cryptococcosis.
